# EUROPEAN PATENT APPLICATION

(11) **EP 4 702 985 A1**
(43) Date of publication of application: **04.03.2026**
(21) Application number: 24020279.6
(22) Date of filing: 30.08.2024
(51) Int. Cl.: A61K 38/07, A61K 38/39, A61P 9/00, C07K 5/10, A61F 2/07, A61L 31/16

(54) **COMPOSITIONS FOR TREATING CARDIOVASCULAR DISEASE**

(71) Applicant: The Boots Company PLC, Nottingham NG2 3AA (GB)
(72) Inventor: BELL, Michael David, Derbyshire, DE55 6EL (GB); BRADLEY, Eleanor Jane, Nottingham, NG9 2ES (GB); SHERRATT, Michael, High Peak, SK23 7SB (GB)
(74) Representative: Mukhtar, Amira

(57) **Abstract**

A composition for use in the treatment of cardiovascular disease in a subject and methods for the same, and a composition suitable for oral administration or parenteral administration, an implantable device comprising said composition, and a method of deploying said implantable device within a subject's blood vessel, the compositions comprising tetrapeptides, peptides, or combinations thereof.

## Description

### Technical Field

The present invention relates to compositions for treating cardiovascular disease in a subject, uses and methods of such compositions, an implantable device comprising such compositions, and a method of deploying said implantable device within a subject's blood vessel.

### Background to the invention

Cardiovascular diseases represent the leading cause of death worldwide, accounting for nearly 18 million deaths annually. The origins of cardiovascular disease are diverse with pathology that can arise from conditions including atherosclerotic vascular disease, obstructive coronary artery disease, myocardial infarction, rhythm abnormalities, and cardiac inflammation.

Existing therapies for heart disease and failure focus on improving cardiac output and efficiency as quickly as possible given the often high risk of death. Few strategies provide longer term provision for homeostatic regulation of extracellular matrix (ECM) production to ensure effective repair following heart trauma. Often this is seen as a secondary or indirect benefit of medications relating to cardiovascular health. Therapeutics impacting the fibrotic response in injuries include angiotensin (AT)-converting enzyme and ATI receptor antagonist, β-blockers, endothelin antagonists, as well as statins.

WO2022106054A1 discloses a peptide combination for use in cosmetic beauty applications, and compositions comprising said peptide combinations. This document teaches the benefits of the peptide combinations in relation to enhancing production of dermal ECM proteins (such as collagen, fibrillin, fibronectin and decorin) in aging skin.

### Summary of the invention

A unifying theme in the progression of many cardiovascular diseases is the development of cardiac fibrosis. The heart responds to injury by producing ECM that includes proteins such as collagen, fibronectin and laminin. Often, however, an overproduction of these ECM components results in fibrosis. Pathological fibrotic remodelling involves changes in myocardial tissue caused by proliferation and activation of resident cardiac fibroblasts (CFs) and alteration of the extracellular matrix (ECM) composition. While structural collagen is essential for maintaining physiological cardiac function, fibrosis represents pathological changes that correspond with worsened clinical outcomes.

Cardiac fibrosis can be reactive, in response to chronic stress (such as inflammation, pressure overload, and ageing) without involving cell death, or reparative. Regardless of the pathological trigger, excessive fibrosis in the heart may have a variety of deleterious consequences and clinical evidence correlates adverse outcomes in patients with heart failure with increased and stiffer cardiac ECM.

The specific peptides and combinations utilised in the present invention enhance production of ECM proteins (such as collagen, fibrillin fibronectin and decorin). The present inventors have identified that these specific peptides and combinations of peptides, previously used as cosmetic skin treatments, are effective to regulate repair of cardiac tissue, preventing excessive fibrosis and scarring and promoting long-term cardiovascular health. The specific peptides and combinations are therefore effective in preventing or treating cardiovascular diseases.

Specifically, the present inventors have discovered that therapeutic interventions that target the ECM offer potential preventative therapies for heart health but also combination therapies post trauma to support reparative processes that reduce fibrosis and scar formation. Not wishing to be bound by theory, this is thought to be because matrikine peptides can act to normalise ECM production ensuring that the reaction to stress is appropriate, and that there is not an excessive over production of ECM proteins that will result in a stiffer myocardial wall and scarring after injury.

In addition to heart tissue itself, matrikine peptides are thought to provide a benefit to the development of fibrotic vessels leading to artery stiffening in ageing or atherosclerosis via the same mechanism, i.e., controlling ECM production and remodelling in response to damage or inflammation.

Cardiovascular disease is characterised by altered ECM composition and quality. Injury and subsequent repair invariably lead to scar tissue formation. Such scar tissue formation can become progressive leading to cardiac fibrosis in which the ECM becomes stiff due to an im-balance in the production of key matrix proteins such as collagen and elastic fibre proteins such as fibrillin. The regulated remodelling of the ECM, especially in healing following a disease, such as a cardiovascular disease, by its resident cells, is a prerequisite for maintenance of tissue homeostasis. In pathological events, qualities of the ECM, such as its composition and topological features, biomechanical properties, or role as a reservoir for secreted mediators, act as instructive cues that affect the behaviour of fibroblasts and other cell types. Once tissue homeostasis is disturbed by repeated injury and repair, aberrant feedback mechanisms between cells and their remodelled ECM trigger a vicious cycle that leads to disease progression.

Within the cardiac interstitium, resident fibroblasts are the most commonly identified cell and are mainly responsible for ECM production; they also serve as effector cells during injury and repair. The ECM produced plays an active role in shaping the behaviour of the fibroblast and other cells. Its quality (based on composition, fibre orientation and stiffness) provides critical cues that help orient cells to their location and provide information about their environment. Both the ECM and the fibroblast are therefore critical components in the early and late repair responses in the cardiovascular system.

The compositions and methods disclosed herein support and promote cardiovascular epithelial ECM repair through production of new collagen and elastic fibre networks. Such ECM repair thereby improves the mechanical properties of the cardiovascular system, long-term cardiovascular health, and decreases susceptibility to future infections and disease.

According to a first aspect of the present invention, there is provided a composition for use in the treatment of a cardiovascular disease in a subject, the composition comprising either a first tetrapeptide, a second tetrapeptide, a third tetrapeptide, a fourth tetrapeptide or a combination of the first tetrapeptide and the second tetrapeptide.

According to a second aspect, the present invention provides a method of treating a cardiovascular disease in a subject, wherein the method comprises administering a composition to the subject, the composition comprising either a first tetrapeptide, a second tetrapeptide, a third tetrapeptide, a fourth tetrapeptide or a combination of the first tetrapeptide and the second tetrapeptide.

In a third aspect there is provided a pharmaceutical composition suitable for oral administration and/or parenteral administration, the composition comprising either a first tetrapeptide, a second tetrapeptide, a third tetrapeptide, a fourth tetrapeptide or a combination of the first tetrapeptide and the second tetrapeptide.

In a fourth aspect, the present invention provides an implantable device comprising:
(i) a framework; and
(ii) a peptide provided on or within the framework such that, in use, the peptide is released into the bloodstream;
wherein the peptide is a first tetrapeptide, a second tetrapeptide, a third tetrapeptide, a fourth tetrapeptide or a combination of the first tetrapeptide and the second tetrapeptide.

Wherein in the first, second, fourth and fifth aspects:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid selected from the group consisting of Valine (V), Aspartic acid (D), Proline (P), Glycine (G) and mixtures thereof, at the N-terminal end, U is selected from the group consisting of H, -CO-R', -SO₂-R¹ or a biotinyl group, at the C-terminal end, Z is selected from the group consisting of OH, O R', NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second tetrapeptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid selected from the group consisting of Lysine (K), Glutamic acid (E) and Serine (S) and mixtures thereof, at the N-terminal end, U is selected from the group consisting of H, -CO-R', - SO₂-R¹ or a biotinyl group, at the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R', -SO₂-R¹ or a biotinyl group. At the C-terminal end, Z is selected from the group consisting of OH, O R', NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, -CO-R¹, -SO₂-R¹ or a biotinyl group. At the C-terminal end, Z is selected from the group consisting of OH, O R', NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a fifth aspect, the present invention provides a method of deploying the implantable device of the fourth aspect within a subject's blood vessel, the method comprising:
- inserting an implantable device according to the fourth aspect, removably coupled to a guide wire into a subject's blood vessel, wherein the implantable device is in a contracted configuration;
- applying force to the guide wire to thereby move the implantable device within the subject's blood vessel to a target location;
- altering the implantable device from a contracted configuration to an expanded configuration; and
- detaching the guide wire from the implantable device and applying force to remove the guide wire from the subject's blood vessel.

### Detailed description of the invention

The present invention is directed toward compositions for use in the treatment of a cardiovascular disease in a subject, and methods of treating a cardiovascular disease in a subject comprising administering a composition to the cardiovascular system of a subject.

The composition and method enable the production of proteins of the cardiovascular epithelial extracellular matrix (ECM), including at least fibrillin, fibronectin, decorin and collagen.

The subject may be a mammal, such as a human, primate, dog, cat, cattle, pig, horse or sheep. In preferred embodiments, the subject is a human. In certain embodiment's, the subject may be a patient.

The cardiovascular system comprises the heart, arteries, veins, and capillaries. In some embodiments, the compositions for use and methods of treatment are directed to the treatment of the heart. In some embodiments, the compositions for use and methods of treatment are directed to the treatment of the arteries. In some embodiments, the compositions for use and methods of treatment are directed towards the treatment of the veins.

The cardiovascular disease may be aneurysm, stable angina, unstable angina, angina pectoris, angioneurotic oedema, aortic valve stenosis, aortic aneurysm, arrhythmia, arrhythmogenic right ventricular dysplasia, artheriosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, carotid stenosis, coronary artery disease, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperaemia, hypertension, pulmonary hypertension, cardiac hypertrophy, left ventricular hypertrophy, right ventricular hypertrophy, hypoplastic left heart syndrome, hypotension, intermittent claudication, ischemic heart disease, Klippel-Trenaunay- Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, Sneddon syndrome, superior vena cava syndrome, syndrome X, tachycardia, Takayasu's arteritis, hereditary haemorrhagic telangiectasia, telangiectasis, temporal arteritis, tetralogy of Fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome. The subject, who may be a patient, may have, have experienced, or be at risk of the above. In other embodiments, the compositions and methods of the present invention may be prophylactic for the above.

In some embodiments the cardiac disease is selected from atherosclerotic vascular disease, obstructive coronary artery disease, myocardial infarction, rhythm abnormalities, cardiac fibrosis or cardiac inflammation.

In some embodiments, the cardiovascular disease is atherosclerotic vascular disease. Atherosclerotic vascular disease relates to exaggerated artery narrowing and stiffening, and can also exacerbate the development of cardiac fibrosis.

In some embodiments, the cardiovascular disease is obstructive coronary artery disease. Obstructive coronary artery disease occurs when plaque builds up within the coronary arteries, leading to artery narrowing, and can also exacerbate the development of cardiac fibrosis.

In some embodiments, the cardiovascular disease is myocardial infarction. Cardiac fibrosis can enhance the likelihood of myocardial infarction, as well as myocardial infarction exacerbating the development of cardiac fibrosis.

In some embodiments, the cardiovascular disease is rhythm abnormalities. Cardiac fibrosis can increase the likelihood of arrhythmias by promoting electrical instability and delayed conduction, as well as rhythm abnormalities exacerbating the development of cardiac fibrosis.

In some embodiments, the cardiovascular disease is cardiac inflammation. Cardiac fibrosis can increase the likelihood of cardiac inflammation, as well as cardiac inflammation exacerbating the development of cardiac fibrosis.

In preferred embodiments, the present invention is directed to compositions for use in method of treating and methods of treating cardiac fibrosis.

### Tetrapeptides

The present invention is directed toward pharmaceutical compositions, compositions for use in the treatment of a cardiovascular disease in a subject, and methods of treating a cardiovascular disease in a subject comprising administering a composition to the cardiovascular system of a subject. The compositions comprise either a first tetrapeptide, a second tetrapeptide, a third tetrapeptide, a fourth tetrapeptide or a combination of the first tetrapeptide and the second tetrapeptide. The invention further provides an implantable device comprising a framework, and a first tetrapeptide, a second tetrapeptide, a third tetrapeptide, a fourth tetrapeptide or a combination of the first tetrapeptide and the second tetrapeptide provided on or within the framework such that, in use, the framework releases the composition.

The first tetrapeptide (a) has generic formulation U-LSXX-Z wherein L is used to denote amino acid Leucine and S is used to denote Serine, as per the internationally recognised single letter code for amino acids. X denotes an amino acid selected from the group consisting of Valine (V), Aspartic acid (D), Proline (P), Glycine (G) and mixtures thereof. At the N-terminal end, U is selected from the group consisting of H, -CO-R', -SO₂-R¹ or a biotinyl group. At the C-terminal end, Z is selected from the group consisting of OH, O R', NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

The tetrapeptide of the present invention is preferably selected from the group consisting of SEQ ID No: 1, SEQ ID No: 2, SEQ ID No: 3, SEQ ID No: 4, SEQ ID No: 5, SEQ ID No: 6, SEQ ID No: 7, SEQ ID No: 8, SEQ ID No: 9, SEQ ID No: 10, SEQ ID No: 11 and SEQ ID No: 12.

In another embodiment of the present invention the tetrapeptide is preferably selected from the group consisting of SEQ ID No: 1, SEQ ID No: 9 and SEQ ID No: 8.

In another embodiment of the present invention the tetrapeptide is preferably selected from the group consisting of SEQ ID No: 1.

In another embodiment of the present invention the tetrapeptide is preferably selected from the group consisting of SEQ ID No: 9.

In another embodiment of the present invention the tetrapeptide is preferably selected from the group consisting of SEQ ID No: 8.

In a preferred embodiment the tetrapeptide combination of the present invention, tetrapeptide a) is selected from the group consisting of U-LSVD-Z, U-LSVP-Z, U-LSVG-Z, U-LSDV-Z, U-LSDP-Z, U-LSDG-Z, U-LSPV-Z, U-LSPD-Z, U-LSPG-Z, U-LSGV-Z, U-LSGD-Z and U-LSGP-Z. In another embodiment, the tetrapeptides is selected from the group consisting of U-LSVD-Z, U-LSPG-Z and U-LSPD-Z. In another embodiment the tetrapeptide a) is Pal-LSVD-OH. In another embodiment the tetrapeptide a) is Pal-LSPG-OH. In another embodiment the tetrapeptide a) is Pal-LSPD-OH.

The second tetrapeptide (b) has generic amino acid sequence U-GPXG-Z wherein G is used to denote amino acid glycine and P denotes the amino acid proline, as per the internationally recognised single letter code for amino acids, X denotes an amino acid selected from the group consisting of Lysine (K), Glutamic acid (E) and Serine (S) and mixtures thereof, at the N-terminal end, U is selected from the group consisting of H, -CO-R', -SO₂-R¹ or a biotinyl group, at the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a preferred embodiment, peptide b) is selected from the group consisting of SEQ ID No: 13, SEQ ID No: 14, SEQ ID No: 15, and SEQ ID No: 16.

In a further preferred embodiment of the present invention peptide b) is U-GPKG-Z.

In a further preferred embodiment of the present invention peptide b) is U-GPEG-Z.

In a further preferred embodiment of the present invention peptide b) is U-GPSG-Z.

The third tetrapeptide (c) has the generic formulation U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R', -SO₂-R¹ or a biotinyl group. At the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

The third tetrapeptide is preferably selected from the group consisting of SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, SEQ ID No: 41, SEQ ID No: 42, SEQ ID No: 43, SEQ ID No: 44, SEQ ID No: 45, SEQ ID No: 46, SEQ ID No: 47, SEQ ID No: 48, SEQ ID No: 49, SEQ ID No: 50, SEQ ID No: 51, SEQ ID No: 52, SEQ ID No: 53 and SEQ ID No: 54.

In another embodiment the third tetrapeptide is preferably selected from the group consisting of SEQ ID No: 25, SEQ ID No: 36, SEQ ID No: 44 and SEQ ID No: 51.

In another embodiment the third tetrapeptide is preferably selected from the group consisting of SEQ ID No: 25.

In another embodiment the third tetrapeptide is preferably selected from the group consisting of SEQ ID No: 36.

In another embodiment the third tetrapeptide is preferably selected from the group consisting of SEQ ID No: 44.

In another embodiment the third tetrapeptide is preferably selected from the group consisting of SEQ ID No: 51.

In a preferred embodiment the third tetrapeptide is selected from the group consisting of U-EKGD-Z, U-ELGD-Z, U-EAGD-Z, U-EIGD-Z, U-ERGD-Z, U-KEGD-Z, U-KLGD-Z, U-KAGD-Z, U-KIGD-Z, U-KRGD-Z, U-LEGD-Z, U-LKGD-Z, U-LAGD-Z, U-LIGD-Z, U-LRGD-Z, U-IEGD-Z, U-IKGD-Z, U-ILGD-Z, U-IAGD-Z, U-IRGD-Z, U-REGD-Z, U-RKGD-Z, U-RLGD-Z, U-RAGD-Z, U-RIGD-Z, U-AEGD-Z, U-AKGD-Z, U-ALGD-Z, U-AIGD-Z and U-ARGD-Z. In another embodiment, the third tetrapeptides is selected from the group consisting of U-EKGD-Z, U-LKGD-Z, U-IRGD-Z and U-AKGD-Z. In another embodiment the third tetrapeptide is U-EKGD-Z. In another embodiment the third tetrapeptide is U-LKGD-Z. In another embodiment the third tetrapeptide is U-IRGD-Z. In another embodiment the third tetrapeptide is U-AKGD-Z.

The fourth tetrapeptide (d) has the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, - CO-R¹, -SO₂-R¹ or a biotinyl group. At the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N

Where, at the N-terminal, U is H then the amino acid is not modified. When, at the C-terminal, Z is OH then the amino acid is not modified. The tetrapeptide is thus not in derivatised form. When other than U is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through vectoring, for example by encapsulation of the peptide.

In a preferred embodiment of the present invention the tetrapeptide is modified at the N-terminal and/or the C-terminal end.

In a preferred embodiment of the present invention, R¹ and/or R² is an alkyl chain of from 1 to 24 carbon atoms, preferably a lipophilic alkyl chain of 3 to 24 carbon atoms.

In a further preferred embodiment of the present invention U is an acyl group -CO-R' and Z is selected from the group consisting of OH, methoxy, ethoxy and NH₂, preferably OH. In a further embodiment, U is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment of the present U is independently selected from lauroyl (C12), myristoyl (C14) and palmitoyl (C16).

In a further preferred embodiment Z is OH and U is independently selected from the group consisting of palmitoyl (C16), myristoyl (C14) and lauroyl (C12). Most preferably U is palmitoyl (C16) and Z is OH.

The tetrapeptides may comprise amino acids in the D- or L- configuration. The tetrapeptides may comprise an acid C-terminus such as -COzH.

The amino acids making up the tetrapeptides according to the invention may be optically pure, be made up of L or D isomers or a mixture thereof. L isomers are those present in the natural state and may be preferred.

The present invention also envisages and includes further derivatives of the tetrapeptide, including for example modification and/or addition of a chemically functional group to one or more of the amino acids but without a change in the carbon skeleton. The present invention also envisages and includes further analogues of the tetrapeptide, including modification and/or addition of a chemically functional group to one or more of the amino acids with a change in the carbon skeletal and complexes of the tetrapeptide with other species such as a metal ion (e.g. copper, zinc, manganese, magnesium, and others).

Tetrapeptides are also envisaged in the form of salts, including hydrochloric salt, or acetate.

The combination of tetrapeptides of the present invention show synergistic benefit in that they offer markedly improved levels of fibrillin-1 production in human fibroblast cells in comparison to the peptides singularly.

### Composition

Compositions of the present invention comprise the above defined tetrapeptides. The tetrapeptides are preferably incorporated into the composition in amount of from 0.10ppm to 10,000ppm, preferably from 0.50ppm to 5,000ppm, more preferably from 1ppm to 1000ppm, and most preferably from 1ppm to 500ppm. In some embodiments the tetrapeptides are incorporated into the composition in an amount of from 0.1ppm to 50ppm. These are again based on a % w/w basis. Thus 100,000ppm is 10% by weight of the composition.

Where the composition comprises a combination of a first tetrapeptide and a second tetrapeptide, the first tetrapeptide and second tetrapeptide may be present in the composition in a weight ratio of from 20:80 to 80:20, such as 30:70 to 80:20, 40:60 to 80:20, 50:50 to 80:20, 60:40 to 80:20, or 70:30 to 80:20, based on the total weight of the first tetrapeptide and a second tetrapeptide. The first tetrapeptide and second tetrapeptide may be present in the composition in a weight ratio of from 20:80 to 80:20, such as 20:80 to 70:30, 20:80 to 60:40, 20:80 to 50:50, 20:80 to 40:60, or 20:80 to 30:70, based on the total weight of the first tetrapeptide and a second tetrapeptide. The first tetrapeptide and second tetrapeptide may be present in the composition in a weight ratio of from 20:80 to 80:20, such as 30:70 to 70:30, or 40:60 to 60:40, based on the total weight of the first tetrapeptide and a second tetrapeptide.

In some embodiments the composition is a pharmaceutical composition. A pharmaceutical composition is a product designed for use by a subject. The pharmaceutical composition may be suitable for oral administration, preferably via alimentary administration, or parenteral administration, preferably by injection into the cardiovascular system such as by intravenous injection.

The composition may further comprise common therapeutic agents used in the treatment of cardiovascular diseases. As such, the composition may comprise the first tetrapeptide and/or second tetrapeptide, and one or more common therapeutic agents.

For example, the composition may comprise two or more common therapeutic agents, such as from 2 to 10, 2 to 5, 1 to 4, or 2 to 3 common therapeutic agents.

The common therapeutic agent may be a cardiac medication.

The cardiac medication may be an anticoagulant, such as apixaban, dabigatran, edoxaban, heparin, rivaroxaban, warfarin, or combinations thereof.

The cardiac medication may be an antiplatelet agent, such as aspirin, clopidogrel, dipyridamole, prasugrel, ticagrelor, or combinations thereof.

The cardiac medication may be an angiotensin-converting enzyme (ACE) inhibitor, such as benazepril, captopril, enalapril, fosinopril, lisinopril, moexipril, perindopril, quinapril, ramipril, trandolapril, or combinations thereof.

The cardiac medication may be an angiotensin II receptor blocker (or inhibitor), such as azilsartan, candesartan, eprosartan, irbesartan, losartan, olmesartan, telmisartan, valsartan, or combinations thereof.

The cardiac medication may be an angiotensin receptor-neprilysin inhibitor (ARNIs), such as sacubitril.

The cardiac medication may be a beta blocker, such as acebutolol, atenolol, betaxolol, bisoprolol, metoprolol, nadolol, propranolol, sotalol, or combinations thereof.

The cardiac medication may be combined alpha and beta-blockers, such as carvedilol, labetalol, or combinations thereof.

The cardiac medication may be a calcium channel blocker, such as amlodipine, diltiazem, felodipine, nifedipine, nimodipine, nisoldipine, verapamil, or combinations thereof.

The cardiac medication may be a cholesterol lowering medication, such as a statin (e.g. atorvastatin, fluvastatin, lovastatin, pitavastatin, pravastatin, rosuvastatin, simvastatin or combinations thereof), a nicotinic acid such as niacin. Cholesterol absorption inhibitor such as ezetimibe, combination statin and cholesterol absorption inhibitors such as ezetimibe/simvastatin, or combinations thereof.

The cardiac medication may be a diuretic, such as acetazolamide, amiloride, bumetanide, chlorothiazide, chlorthalidone, furosemide, hydro-chlorothiazide, indapamide, metalozone, spironolactone, torsemide, or combinations thereof.

The cardiac medication may be a vasodilator, such as isosorbide dinitrate, isosorbide mononitrate, hydralazine, nitroglycerin, minoxidil, or combinations thereof.

The cardiac medication may be selected from growth factors and cytokines, such as VEGF (Vascular endothelial derived growth factor), IGF-1 (Insulin Growth Factor), HGF (Hepatocyte Growth Factor), NRG-1 (Neuroregulin), EGF (Epidermal Growth Factor), FGF (Fibroblast Growth Factor), and TGFβ (Transforming Growth Factor) that to induce cardiac repair.

The composition of the present invention may be aqueous or non-aqueous and comprise of a single-phase system or multiple phase system. Single-phase systems include aqueous and non-aqueous compositions. Multiphase systems include but are not limited to microemulsions, emulsions, and products with discrete separate phases. Emulsions include water-in-oil, oil-in-water emulsions and multiple emulsions (water in oil in water or oil in water in oil for example). The skilled person would be capable of formulating the composition such that it is suitable for oral administration or parenteral administration. Examples of methods of preparing a composition suitable for oral administration and a composition suitable for parenteral administration are included below.

Administration of the composition may be by any suitable method. For example, administration of the compositions may be by oral or parenteral administration.

Methods of oral administration include buccal administration, sublabial administration, sublingual administration, or ingestion, The form of the composition for oral administration may be an oral dosage form, such as tablets, capsules, liquids, lozenges, films, powders, pastes and the like.

Administration may be via parenteral administration, such as administration by injection or infusion. Examples of injection include intradermal injection, subcutaneous injection, intramuscular injection, intravenous injection, or intracardial injection.

### Methods of use

The present invention also relates to a method of treating a cardiovascular disease in a subject. The method comprises administering to said subject the composition (e.g. in a therapeutically effective amount) as defined herein.

The method may comprise introducing the composition to the cardiovascular system of the subject, for example by introducing the composition to the bloodstream.

The method may comprise administration of the composition by any suitable route of administration. The method may comprise oral administration, preferably via alimentary administration, or parenteral administration, preferably by injection into the cardiovascular system such as by intravenous injection.

### Composition suitable for oral administration

The present invention also relates to compositions suitable for oral administration. The composition may be provided in an oral dosage form suitable for oral administration according to methods and techniques known in the art.

For example, in some embodiments the oral dosage form may further comprise a binder, such as hydroxypropyl cellulose, microcrystalline cellulose, polyvinylpyrrolidone, gelatine, polyethylene glycol, glycerylbehenate, glycerylmonostearate, carnauba wax, starch, carbomers, sodium carboxymethyl cellulose, or combinations thereof.

In some embodiments, the oral dosage form may further comprise a disintegrant, such as calcium alginate, calcium sodium alginate, calcium carboxymethylcellulose, calcium cellulose glycolate, carmellose calcium, microcrystalline cellulose, powdered cellulose, chitosan hydrochloride, corn starch, pregelatinized starch, crospovidone, low-substituted hydroxypropyl cellulose, hydroxypropyl starch, magnesium aluminum silicate, methylcellulose, sodium alginate, starch, sodium starch glycolate, croscarmellose sodium, or combinations thereof.

In some embodiments, the oral dosage form may further comprise a coating, such as hydroxypropylmethylcellulose, hydroxypropylcellulose, titanium dioxide, calcium carbonate, polyvinyl acetate, talc, sweeteners, colorants, or combinations thereof. The coating may be an enteric coating, and may refer to a polymer barrier that prevents dissolution of the oral dosage form in the gastric environment. Common polymer barriers include anionic polymethacrylates, cellulose based polymers (e.g. cellulose acetate phthalate), and polyvinyl derivatives (e.g. polyvinyl acetate phthalate).

In some embodiments, the oral dosage form may further comprise a lubricant, such as starch, corn starch, talc, silica, colloidal silica, tri-basic calcium phosphate, magnesium silicate, calcium stearate, stearic acid, talc, sodium lauryl sulfate, magnesium lauryl sulfate, sodium stearyl fumarate, magnesium stearate, or combinations thereof.

In some embodiments, the oral dosage form may further comprise a plasticizer, such as glycerol, PEG400, PEG1000, triacetin, or combinations thereof.

In some embodiments, the oral dosage form may further comprise a filler, such as microcrystalline cellulose, powdered cellulose, mannitol, lactose, calcium phosphate, starch, pre-gelatinized starch, or combinations thereof.

### Composition suitable for parenteral administration

The present invention also relates to compositions suitable for parenteral administration. The composition may be provided in an injectable composition suitable for parenteral administration according to methods and techniques known in the art. Excipients used in parenteral products are well known in the art.

In some embodiments the injectable composition may further comprise a pharmaceutically acceptable carrier, such as water.

In some embodiments, the injectable composition may further comprise a buffering agent, such as acetates, bicarbonates, carbonates, citrates, and phosphates, or combinations thereof.

In some embodiments, the injectable composition may further comprise a solubilizing agent, such as watersoluble organic solvents, non-ionic surfactants, water-insoluble lipids, cyclodextrins, or combinations thereof.

In some embodiments, the injectable composition may further comprise a preservative, such as phenol alcohol, benzyl alcohol, or combinations thereof.

In some embodiments, the injectable composition may further comprise a viscosity modifier, such as hydrocolloid gums, vinyl polymers, aluminium silicates, carbomers, or combinations thereof.

In some embodiments, the injectable composition may further comprise a tonicity adjuster, such as sodium chloride, dextrose, mannitol, potassium chloride, or combinations thereof.

In some embodiments, the injectable composition may further comprise a complexing agent, such as ethylenediaminetetraacetic acid (EDTA), tannic acid, DL-tartaric acid, or combinations thereof.

In some embodiments, the injectable composition may further comprise an antioxidant, such as vitamin C, sodium metabisulfite, or combinations thereof.

In some embodiments, the injectable composition may further comprise a surfactant, such as polyethylene glycol sorbitan monooleate, sorbitan oleate, sodium lauryl sulfate, polyvinyl alcohol (PVA), methylcellulose, polyvinylpyrrolidone, lecithin, gelatin, polyoxyethylene sorbitan fatty acid esters, polyoxyethylene castor oil derivatives, sodium stearate, ester amines, linear diamines, fatty amines, or combinations thereof.

In some embodiments, the injectable composition may further comprise a solvent, such as acetone, acetonitrile, benzene, butyl alcohol carbon disulfide, carbon tetrachloride, chloroform, cyclohexane, 1,1-dichloroethane, dimethoxyethane, ethanol, diethyl ether, ethyl acetate, heptane, hexane, methanol, methyl acetate, methyl t-butyl ether, pentane, propyl alcohol, tetrahydrofuran, or combinations thereof. Alternatively, the solvent may be cyclohexane, pentane, hexane, heptane, carbon tetrachloride, carbon disulfide, benzene, diethyl ether, methyl t-butyl ether, tetrahydrofuran, ethyl acetate, and methyl acetate, chloroform, or combinations thereof.

### Implantable Device

The present invention also relates to an implantable device comprising:
(i) a framework; and
(ii) a peptide provided on or within the framework such that, in use, the peptide is released into the bloodstream;
wherein the peptide is the first tetrapeptide, the second tetrapeptide, the third tetrapeptide, the fourth tetrapeptide or a combination of the first tetrapeptide and the second tetrapeptide, as defined above.

The framework may comprise a plurality of interweaved wires that provide a tubular section. The framework may have a contracted configuration wherein the tubular section has a first diameter. The first diameter may be selected such that the tubular section passes through the artery or a vein of a subject. The framework may have an expanded configuration wherein the tubular section has a second diameter than is greater than the first diameter of the contracted configuration. The second diameter may be from 1 to 6 mm, such as from 2 to 5 mm.

The peptide is provided on or within the framework such that, in use, the framework releases the peptide. In an embodiment, the peptide combined with or comprise within a carrier matrix, such as a polymer carrier matrix, which is provided on or within the framework. The carrier matrix may be coated on the framework. The carrier matrix may be embedded within the framework.

In some embodiments, the polymer carrier matrix is a nonbiodegradable polymer such as Parylene C. polybutylmethacrylate (PBMA), polyethylenevinyl acetate (PEVA), polystyrene-block-isobutylene-block-styrene (SIBS), polycarbonate (PC), polyvinylidene fluoride-co-hexafluoropropylene (PVDF-HFP), or Biolinks ^{®}. The carrier matrix may be a biodegradable polymer such as polylactic acid (PLA), poly-L-lactide-co-D,L-lactide (PDLLA), polyglycolic acid (PGA), polylactic-co-glycolic acid (PLGA), or poly (lactide-co-ethylene carbonate) (PLEC). The skilled person would be aware of other exemplary nonbiodegradable polymers and biodegradable polymers that may be used in drug eluting stent platforms.

In an embodiment, the implantable device is an intravascular implantable device, and the framework is a stent platform. For example, the stent platform may be a bare metal stent platform or a drug eluting stent platform. In one embodiment, the stent platform is a drug eluting stent platform, such as a durable polymer drug-eluting stent platform or bioabsorbable polymer drug-eluting stent platform. Where the stent platform is a drug eluting stent platform, the polymer may be comprised within the drug eluting stent platform.

In an embodiment, the peptide is provided on or within the framework using a carrier matrix, such as a polymer carrier matrix. The carrier matrix may be coated on the framework. The carrier matrix may be embedded within the framework. The polymer carrier matrix may be a nonbiodegradable polymer such as Parylene C, polybutylmethacrylate (PBMA), polyethylenevinyl acetate (PEVA), polystyrene-block-isobutylene-block-styrene (SIBS), polycarbonate (PC), polyvinylidene fluoride-co-hexafluoropropylene (PVDF-HFP) The carrier matrix may be a biodegradable polymer such as polylactic acid (PLA), poly-L-lactide-co-D,L-lactide (PDLLA), polyglycolic acid (PGA), polylactic-co-glycolic acid (PLGA), or poly (lactide-co-ethylene carbonate) (PLEC). The skilled person would be aware of other exemplary nonbiodegradable polymers and biodegradable polymers that may be used in drug eluting stent platforms.

In an embodiment, the polymer carrier matrix, in use, releases the composition from the matrix. In use, the polymer carrier matrix may release the entirety of the composition immediately. In use, the polymer carrier matrix may release the composition gradually over time, thereby providing a sustained release of the composition.

The sustained release may be from one hour to one year, such as from one hour to six months, from one hour to three months, from one hour to one month, from one hour to two weeks, or from one hour to one week. The skilled person would be aware of the materials required to achieve the required sustained release.

In an embodiment, the implantable device is implantable within a blood vessel. In an embodiment, the implantable device is implantable within an artery, such as a coronary artery. In an embodiment, the implantable device is implantable within a vein.

The present invention also relates to a method of deploying an implantable device within a subject's blood vessel. The method comprises:
- inserting an implantable device according to the present invention, removably coupled to a guide wire into a subject's blood vessel, wherein the implantable device is in a contracted configuration;
- applying force to the guide wire to thereby move the implantable device within the subject's blood vessel to a target location;
- altering the implantable device from a contracted configuration to an expanded configuration;
- detaching the guide wire from the implantable device and applying force to remove the guide wire from the subject's blood vessel.

The skilled person is aware of methods of deploying implantable devices within a subject's blood vessel.

By way of example, where the implantable device is a drug eluting stent the method may comprise:
- providing the patient with a local or general anaesthetic;
- selecting a site to access the vascular system, such as the femoral artery in the groin or the radial artery in the wrist, and making an incision at this site for insertion of a sheath into the artery to allow vascular access;
- inserting a guide catheter through the sheath and advancing to the coronary arteries to provide access for a contrast dye facilitating visualisation of the coronary arteries via X-ray to be injected;
- mounting the implantable device of the present invention, which is a drug eluting stent, on to a balloon catheter using techniques known in the art, deflating the balloon catheter, and advancing the implantable device through the guide catheter to the site of arterial constriction or blockage;
- inflating the balloon catheter once the implantable device reaches the target area such that the implantable device, which is a drug-eluting stent; is pressed against the arterial wall and released from the balloon catheter;
- deflating and then withdrawing the balloon catheter;
- removing the balloon catheter and guide catheter from the vascular system and incision, and closing the incision.

The target location may be the section of the blood vessel where prevention of narrowing is desired.

Altering the implantable device from a contracted configuration to an expanded configuration may require an expansion means. The expansion means may be provided within the tubular section of the implantable device and coupled to the guide wire. The expansion means may be a balloon. The expansion means may be expanded by inflation to push against the tubular section of the implantable device to thereby alter the configuration from contracted to expanded. Following altering the implantable device from a contracted configuration to an expanded configuration, the expansion means may be deflated and removed from the subject's blood vessel using the guide wire.

The blood vessel may be an artery or a vein.

### Detailed Description of Drawings

Embodiments of the present invention will now be described by way of example with reference to the accompanying drawings, in which:
**Figure 1** is a cross section view of an implantable device of the present invention.
**Figure 2** is a flow diagram of the method of deploying an implantable device according to the present invention.

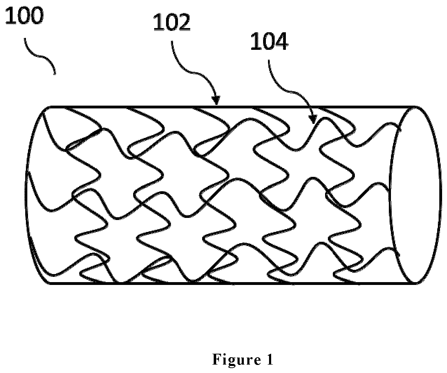

Referring first to Figure 1 of the accompanying drawings, there is shown according to the present invention an implantable device 100 comprising a framework 102 and a peptide provided on or within the framework (not shown). The framework of the example comprises a plurality of interweaved wires 104 of the framework 102.

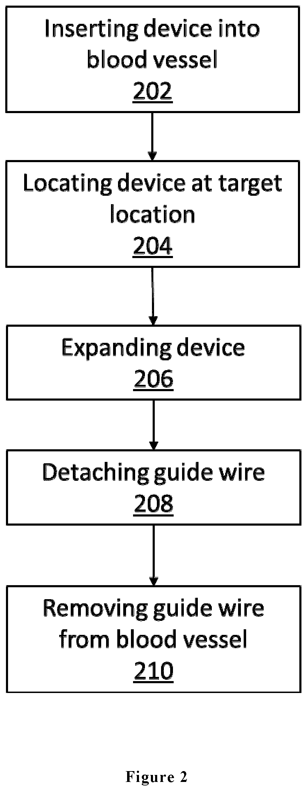

Referring now to Figure 2, there is shown a flow diagram of the method of deploying the implantable device within a subject's blood vessel according to the present invention. The method comprises in step 202 inserting an implantable device according to the present invention, removably coupled to a guide wire into a subject's blood vessel, wherein the implantable device is in a contracted configuration; in step 204 applying force to the guide wire to thereby move the implantable device within the subject's blood vessel to a target location; in step 206 altering by expansion the implantable device from a contracted configuration to an expanded configuration; in step 208 detaching the guide wire from the implantable device and in step 210 applying force to remove the guide wire from the subject's blood vessel.

### Examples

The present invention is further described by the following examples.

### Examples of tetrapeptide synthesis

The tetrapeptides of the present invention with generic formulation pal-X¹X²X³X⁴-OH are prepared by peptidic synthesis. In a first step the N-terminal of X⁴ is coupled with a resin via the terminal acid functionality in the presence of a coupling agent. The N terminal amine is then reacted with the next amino acid in the sequence X³ in the presence of a coupling agent. The same process is repeated until the required sequence is obtained and a suitable C terminal functionality added. Suitable coupling agents include DCC (dicyclohexylcarbodiimide)/NHS (N-hydroxysuccinimide) or HBTU (2-(1H-benzotriazole-1-yl)-1,1,3,3-tetramethyluronium hexafluorophosphate)/HOBT (1-hydroxy-benzotriazole)). The resulting peptide is then cleaved from the resin in an acidic medium and after precipitation, washing and drying, the peptide is obtained in solid form.

### Example Compositions

### Oral Tablet

| **Ingredient** | **Concentration/weight (per 250mg tablet)** |
|---|---|
| Ezetimibe | 10mg per tablet |
| Simvastatin | 10mg per tablet |
| lactose monohydrate (excipient) | 125 mg per tablet |
| Tetrapeptide Pal-LSVD | 0.0125mg per tablet (40ppm) |
| Tetrapeptide Pal-GPKG | 0.0125mg per tablet (40ppm) |
| Magnesium stearate (excipient) | 2mg per tablet |
| Microcystalline cellulose (excipient) | 102.975mg per tablet |

### Method of Manufacture

1. The active ingredients Simvastatin, Ezetimibe and the tetrapeptides are accurately weighed out and dispensed into clean, labeled containers in a controlled environment to avoid contamination.
2. The active ingredients are then mixed in a large blender with the excipient materials lactose monohydrate and microcrystalline cellulose until they are uniformally distributed.
3. Magnesium stearate is then added as a lubricant to prevent sticking during tablet compression. and mixed thoroughly.
4. The powder is then compacted into large slugs using a roller compactor and milled into granules
5. Finally, the granules are fed into a tablet press machine, where they are compressed into tablets. The press applies high pressure to form the tablets and imprint necessary markings (e.g., dosage, brand name).

### Arterial Stent

| **Ingredient** | **Weight (Total weight of stent 30mg)** |
|---|---|
| Cobalt chromium alloy | 25mg |
| Poly (lactic-co-glycolic acid) PGLA | 5mg |
| Anti-proliferative drug Sirolimus | 1mg |
| Anti-thrombotic ingredients clopidogrel | 1mg |
| Tetrapeptide Pal-LSVD | 0.001mg per stent (33ppm) |
| Tetrapeptide Pal-GPKG | 0.001mg per stent (33ppm) |

1. A thin metal tube is cut from the cobalt chromium alloy into a specific stent pattern using precision laser cutting, allowing the stent to be inserted into the artery.
2. After cutting, the stent undergoes electropolishing to smooth the surface and remove any burrs or sharp edges, reducing the risk of injury to the artery during deployment.
3. The stent is then crimped onto a balloon catheter to reduce its diameter and allow it to be inserted into the artery.
4. The PLGA is used as a coating and reservoir for the therapeutic agents including the peptides. The polymer is dissolved in solvent to create a coating solution before the agents (Sirolimus, clopidrogen and the tetrapeptides) are mixed into the solution.
5. The stent is then coated with the polymeric-drug mixture by spraying in a controlled environment to ensure optimum film thickness and uniformity.
6. The stent is then cured in an oven to evaporate all solvent and solidify the polymer layer.

## Claims

1. A composition for use in the treatment of a cardiovascular disease in a subject, the composition comprising either a first tetrapeptide, a second tetrapeptide, a third tetrapeptide, a fourth tetrapeptide or a combination of the first tetrapeptide and the second tetrapeptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R', -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second tetrapeptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysin(K), Glutamic acid (E), Proline (P) and Serine (S) and mixtures thereof, at the N-terminal end, U is selected from the group consisting of H, -CO-R', -SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R', -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

2. A method of treating a cardiovascular disease in a subject, wherein the method comprises administering a composition to the subject, the composition comprising either a first tetrapeptide, a second tetrapeptide, a third tetrapeptide, a fourth tetrapeptide or a combination of the first tetrapeptide and the second tetrapeptide, wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R', -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second tetrapeptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, at the N-terminal end, U is selected from the group consisting of H, -CO-R', -SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R', -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

3. The composition for use of claim 1, or the method of use of claim 2, wherein the composition is administered via oral administration or parenteral administration

4. A pharmaceutical composition suitable for oral administration and/or parenteral administration, the composition comprising either a first tetrapeptide, a second tetrapeptide, a third tetrapeptide, a fourth tetrapeptide or a combination of the first tetrapeptide and the second tetrapeptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
b) the second tetrapeptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, at the N-terminal end, U is selected from the group consisting of H, -CO-R', -SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R', -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

5. The composition for use of any one of claims 1 or 3, the method of use of any one of claims 2 or 3, or the composition of claim 4 wherein the first tetrapeptide a) is selected from the group consisting of
i) SEQ ID No: 1, SEQ ID No: 2, SEQ ID No: 3, SEQ ID No: 4, SEQ ID No: 5, SEQ ID No: 6, SEQ ID No: 7, SEQ ID No: 8, SEQ ID No: 9, SEQ ID No: 10, SEQ ID No: 11 and SEQ ID No: 12, preferably the group consisting of SEQ ID No: 1, SEQ ID No: 9 and SEQ ID No: 8; or
ii) U-LSVD-Z, U-LSPG-Z, and U-LSPD-Z.

6. The composition for use, the method of use, or the composition of any preceding claim wherein the second peptide b) is selected from the group consisting of
i) SEQ ID No: 13, SEQ ID No: 14, SEQ ID No: 15, and SEQ ID No: 16; or
ii) U-GPKG-Z, U-GPEG-Z and U-GPSG-Z.

7. The composition for use, the method of use, or the composition of any preceding claim wherein U of the tetrapeptide is independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle, preferable the group consisting of lauroyl (C12), myristoyl (C14) and palmitoyl (C16).

8. The composition for use, the method of use, or the composition of any preceding claim wherein the third tetrapeptide (c) is selected from the group consisting of
i) SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, SEQ ID No: 41, SEQ ID No: 42, SEQ ID No: 43, SEQ ID No: 44, SEQ ID No: 45, SEQ ID No: 46, SEQ ID No: 47, SEQ ID No: 48, SEQ ID No: 49, SEQ ID No: 50, SEQ ID No: 51, SEQ ID No: 52, SEQ ID No: 53 and SEQ ID No: 54, preferably from the list consisting of SEQ ID No: 25, SEQ ID No: 36, SEQ ID No: 44 and SEQ ID No: 51; or
ii) Pal-AKGD-OH, Pal-EKGD-OH, Pal-LKGD-OH, and Pal-IRGD-OH.

9. The composition for use, the method of use, or the composition of any preceding claim, wherein a peptide is present at from 0.1ppm to 10,000ppm by weight of the composition.

10. The composition for use, the method of use, or the composition of any preceding claim, wherein the first tetrapeptide a) and second tetrapeptide b) are present in the composition in a weight ratio of from 20:80 to 80:20 based on the total weight of tetrapeptides a) and b).

11. An implantable device comprising:
(i) a framework; and
(ii) a peptide provided on or within the framework such that, in use, the peptide is released into the bloodstream;
wherein the peptide is a first tetrapeptide, a second tetrapeptide, a third tetrapeptide, a fourth tetrapeptide or a combination of the first tetrapeptide and the second tetrapeptide; and wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R', -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second tetrapeptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, at the N-terminal end, U is selected from the group consisting of H, -CO-R', -SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R', -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

12. The implantable device of claim 11, wherein the implantable device is an intravascular implantable device, and the framework is a stent platform, optionally a drug-eluting stent platform.

13. The implantable device of any one of claims 11 or 12, wherein the composition is provided on or within the framework using a carrier matrix, and wherein the carrier matrix, in use, releases the composition.

14. The implantable device of any one of claims 11-13, wherein the implantable device is implantable within a blood vessel, wherein the blood vessel is:
a) an artery, such as a coronary artery; or
b) a vein.

15. A method of deploying an implantable device within a subject's blood vessel, the method comprising:
• inserting an implantable device of any one of claims 16-20, removably coupled to a guide wire into a subject's blood vessel, wherein the implantable device is in a contracted configuration;
• applying force to the guide wire to thereby move the implantable device within the subject's blood vessel to a target location;
• altering the implantable device from a contracted configuration to an expanded configuration;
• detaching the guide wire from the implantable device and applying force to remove the guide wire from the subject's blood vessel.
